# EUROPÄISCHE PATENTSCHRIFT

(11) **EP 2 111 892 B1**
(45) Veröffentlichungstag und Bekanntmachung des Hinweises auf die Patenterteilung: **16.04.2014**
(21) Anmeldenummer: 09156052.4
(22) Anmeldetag: 24.03.2009
(51) Int. Cl.: A61N 1/362

(54) **Antitachykarder Herzstimulator**
Anti-tachycardic heart stimulator
Stimulateur cardiaque contre la tachychardie

(30) Priorität: 22.04.2008 DE 102008020022
(43) Veröffentlichungstag der Anmeldung: 28.10.2009
(73) Patentinhaber: Biotronik CRM Patent AG, 6341 Baar (CH)
(72) Erfinder: Dörr, Thomas, 12437, Berlin (DE); Schneider, Peter, 12527, Berlin (DE); Lang, Torsten, 12437, Berlin (DE)
(74) Vertreter: Lindner-Vogt, Karin L.

(56) Entgegenhaltungen:
- WO-A1-2005/107865
- US-A1- 2004 106 956
- US-A1- 2006 217 769
- US-B2- 6 889 080

## Beschreibung

Die Erfindung betrifft einen implantierbaren Herzstimulator für die Therapie tachykarder Herzrhythmusstörungen. Ein solcher Herzstimulator ist auch als implantierbarer Cardioverter/Defibrillator (ICD) bekannt und erfüllt in der Regel auch die Funktion eines implantierbaren Herzschrittmachers.

Der hier betroffene implantierbare Herzstimulator umfasst eine Vorrichtung gemäß Anspruch 1.

Stimulations- und Sensingelektroden können dabei miteinander identisch sein und abwechselnd zur Abgabe von Stimulationsimpulsen und zur Aufnahme von Myocard-Potentialen dienen.

Unter einer Tachyarrhythmie versteht man eine Herzrhythmusstörung, die zu einer höheren Herzrate führt, als physiologisch angemessen ist. Man unterscheidet zum einen Tachykardien und Fibrillationen. Während bei einer Tachykardie (Herzflattern) noch eine Kontraktion einer betroffenen Herzkammer erfolgt, zeichnet sich eine Fibrillation (Herzflimmern) dadurch aus, dass die betroffene Herzkammer einer umlaufenden Erregung ausgesetzt ist, bei der sich Teile des Herzmuskelgewebes (Myokards) der Herzkammer bereits wieder entspannen, während andere Teile erst kontrahieren, so dass zu keiner periodischen Kontraktion und Entspannung der Herzkammer mit für das Pumpen erforderlicher Volumenänderung kommt. Innerhalb der Tachykardien werden u.a. ventrikuläre Tachykardien (VT) von superventrikulären Tachykardien (SVT) unterschieden, die beide Tachyarrhythmien einer oder beider Herzkammern (rechter und/oder linker Ventrikel) sind. Während superventrikuläre Tachykardien ihren Ursprung im Atrium haben und durch atrioventrikuläre Reizleitung auf den jeweiligen Ventrikel übergeleitet werden, haben ventrikuläre Tachykardien ihren Ursprung im jeweiligen Ventrikel selbst.

Derzeit bieten alle Hersteller implantierbarer Cardioverter/Defibrillatoren (ICD) Geräte an, die zur Diskriminierung zwischen ventrikulärer (VT) und supraventrikulärer Therapie (SVT) in der Lage sind. Eine Gemeinsamkeit dieser Geräte ist es immer, dass sie vor einer Therapie einer Tachyarrhythmie den Ursprung der Tachyarrhythmie festzustellen und - abhängig von dessen Ursprung - eine entsprechende Therapie einzuleiten.

Bekannte Ansätze zur Unterscheidung zwischen ventrikulärer und supraventrikulärer Tachykardie bestehen darin, z.B. unregelmäßige, schnelle atriale und ventrikuläre Rhythmen einer VT zuzuordnen und regelmäßige schnelle Rhythmen hingegen einer SVT, siehe z. B. US 5,686,793, US 5,891,170, US 6,748,269, US 6,889,080, US 7,174,209 und US 2004/0093037. Auf diese Weise erfolgt eine Diskriminierung (Unterscheidung) zwischen VT und SVT vor Einleitung einer entsprechenden Therapie.

Keiner der derzeit verfügbaren Geräte verfügt dabei über eine 100%ige Spezifität, d. h. inadäquate Therapieabgaben sind die Folge. Besonders kritisch sind in diesem Zusammenhang schmerzhafte Schockabgaben anzusehen, da diese die Akzeptanz der ICD-Therapie drastisch verschlechtern.

Nicht etabliert haben sich sog. "aktive" Diskriminierungsalgorithmen, die mittels gezielter ventrikulärer oder atrialer Stimulation und Beobachtung der jeweils benachbarten Kammer (Atrium oder Ventrikel) das Diskriminierungsergebnis verbessern können. Wesentlicher Nachteil sind hier die mit der Stimulationsphase verbundene Verzögerung der Detektionsentscheidung und das proarrhythmische Potential einer solchen Stimulation im Vorfeld der Detektionsentscheidung.

Die Aufgabe der Erfindung besteht darin, inadäquate Schocktherapien im Falle von Herzrhythmen, die einer ventrikulären Tachykardie zugeordnet werden, d.h. in eine zuvor festgelegte VT-Zone fallen, zu verhindern. Dabei soll jedoch eine notwendige (adäquate) ventrikuläre Therapie gegenüber den derzeitigen Algorithmen möglichst nicht zusätzlich verzögert werden.

Erfindungsgemäß wird die Aufgabe durch einen implantierbaren Herzstimulator gemäß Anspruch 1 gelöst.

Ein ähnliches Konzept, das jedoch nicht auf die Analyse atrialer Rhythmusmuster abstellt, ist aus US 7,149,577 bekannt. In US 7,149,577 wird die Analyse der Rhythmus vor und nach einer Stimulation in dem Kanal beschrieben, in dem auch die Stimulation selbst erfolgt. Im Unterschied dazu erfolgt hier die Analyse des Rhythmus grundsätzlich in einem anderen Kanal als dem stimulierten (speziell im Atrium bei ventrikulärer ATP). Ferner ist es nun möglich, die Rhythmusanalyse auch während der Stimulation durchzuführen. Dies ist in US 7,149,577 prinzipiell ausgeschlossen.

Das Dokument WO-A-2005/107865 offenbart eine Vorrichtung gemäß Oberbegriff des Anspruchs 1.

Die dabei zugrundeliegende Hypothese ist die, dass bei gleichzeitig schnellem Rhythmus in Atrium und Ventrikel der atriale Rhythmus bei einer SVT nicht durch eine ventrikuläre Stimulation in seinem Rhythmusmuster beeinflusst wird, bei einer "echten" VT jedoch durch die ventrikuläre Stimulation moduliert wird. Die derzeitigen Algorithmen zur VT/SVT-Diskriminierung unterscheiden hingegen nicht zwischen der nachfolgenden Therapie (ATP-schmerzlos / Schock-schmerzhaft) und beziehen auch nicht die Informationen, die während der Therapieabgabe verfügbar sind, in die Therapieentscheidung mit ein. Die Auswerteeinheit stellt somit eine Art zweite Tachykardiediskriminierungseinheit dar.

Der Herzstimulator ist vorzugsweise ein antitachykarder Stimulator mit mindestens einer VT- und einer VF-Zone, d.h. tachykarde Herzraten bis zur einer Grenzrate, die eine obere Grenze der VT-Zone darstellt, werden einer Tachykardie zugeordnet und Herzraten oberhalb dieser Grenzrate, die gleichzeitig eine untere Grenze der VF-Zone darstellt, werden einer ventrikulären Fibrillation zugeordnet. Mit Zone sind somit Bereiche von Herzraten gemeint, die im Falle der VT-Zone einer Tachykardie und im Falle der VF-Zone einer Fibrillation zugeordnet werden. Das Konzept verschiedener Zonen ist aus dem Stand der Technik, beispielsweise aus US 5,144,947 oder US 2004/0093037, grundsätzlich bekannt.

Weiterhin ist der Herzstimulator vorzugsweise ein antitachykarder Stimulator mit einer Vorrichtung zur Abgabe mindestens eines Defibrillationsschocks, d.h. mit einem Defibrillationsschockgenerator, der mit einer Defibrillationsschockelektrode verbunden oder zu verbinden und ausgebildet ist, einen Defibrillationsschock zur Defibrillation wenigstens einer Herzkammer zu generieren und auszugeben.

Die Therapiesteuereinheit kann hierbei ausgebildet sein, einen Defibrillationsschock in Abhängigkeit des ATP-Antwortsignals zu inhibieren, und zwar vorzugsweise nur, wenn die ventrikuläre Herzrate in der VT-Zone, nicht aber in einer VF-Zone liegt.

Die Auswerteeinheit kann dazu ausgebildet sein, den atrialen Rhythmus unmittelbar nach einer ATP und im Vergleich mit dem atrialen Rhythmus unmittelbar vor der ATP zu bewerten.

Folgende weitere Varianten einer Therapieablaufsteuerung durch die Therapiesteuereinheit sind vorteilhaft:
- In Abhängigkeit des ATP-Anwortsignals wird die nachfolgende antitachykarde Therapie inhibiert.
- In Abhängigkeit des ATP-Anwortsignals wird die nachfolgende antitachykarde Therapie fortgesetzt.
- In Abhängigkeit des ATP-Anwortsignals wird die nachfolgende antitachykarde ventrikuläre Therapie durch eine atriale antitachykarde Therapie ersetzt.
- In Abhängigkeit des ATP-Anwortsignals wird zunächst eine neue VT/SVT-Klassifikation (Tachykardiediskriminierung) gestartet.
- In Abhängigkeit des ATP-Anwortsignals werden die Parameter der ventrikulären antitachykarden Therapie (ATP) angepasst.

Die erfindungsgemäße Lösung bietet den Vorteil, dass die Spezifität der VT/SVT-Diskriminierung weiter erhöht werden kann und damit die Zahl der inadäquaten Schocktherapien reduziert wird. Diese Reduzierung führt zu einer gesteigerten Akzeptanz der ICD-Therapie. Diese Diskriminierungsverbesserung führt dabei zur keiner Verlängerung der Detektionszeit für die VT-Detektion.

Die Erfindung soll nun anhand eines Ausführungsbeispiels mit Bezug auf die Figuren näher erläutert werden. Von den Figuren zeigt:
- Fig. 1:: einen implantierbaren Dreikammer-Kardioverter/Defibrillator als beispielhaften Herzstimulator in Verbindung mit daran angeschlossenen Elektrodenleitungen;
- Fig. 2:: ein schematisches Blockschaltbild des Herzstimulators aus Figur 1;
- Fig. 3:: einige Komponenten des Herzstimulators in detaillierterer Darstellung;
- Fig. 4:: eine erste Implementierung einer Therapiesteuerung in Reaktion auf ein ATP-Antwortsignal in Form eines Flussdiagramms;
- Fig. 5:: eine zweite Implementierung einer Therapiesteuerung in Reaktion auf ein ATP-Antwortsignal in Form eines Flussdiagramms; und

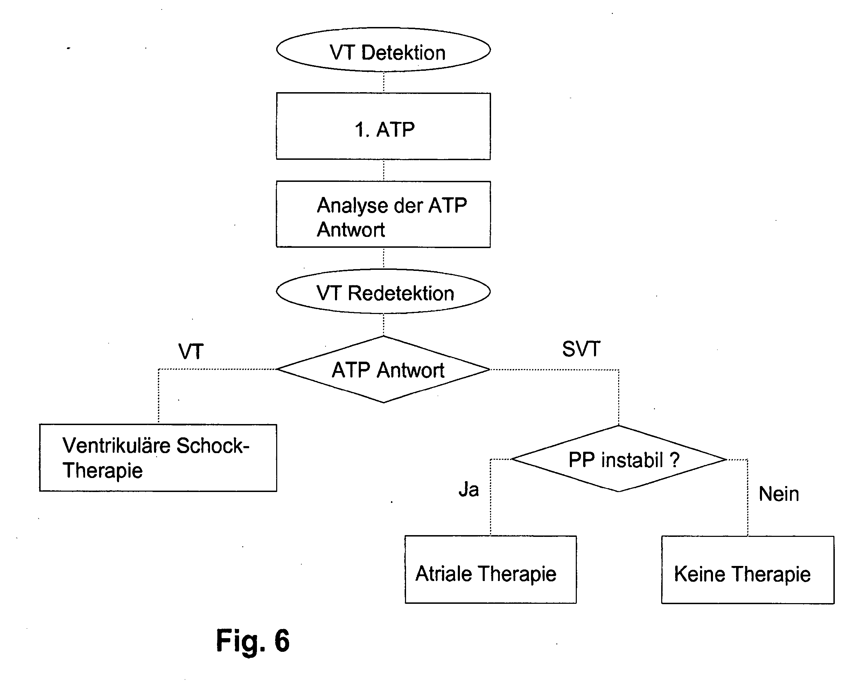

- Fig. 6:: eine dritte Implementierung einer Therapiesteuerung in Reaktion auf ein ATP-Antwortsignal in Form eines Flussdiagramms.

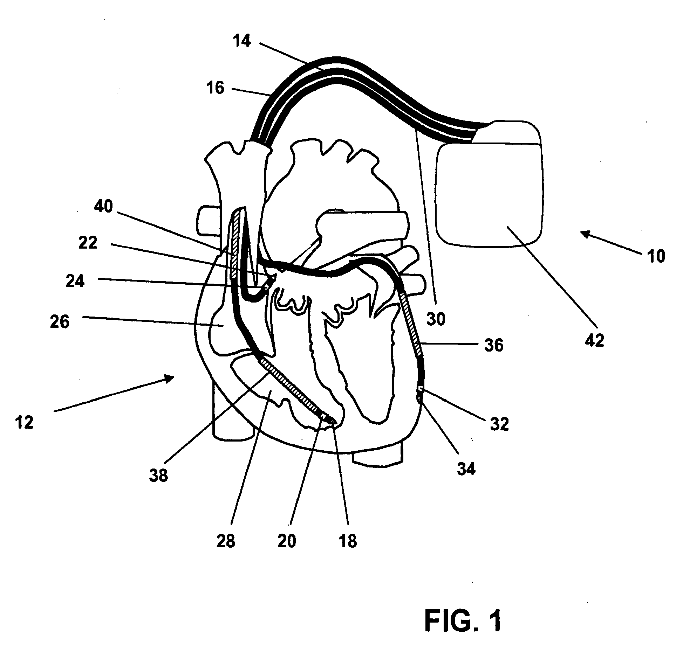

Fig. 1 zeigt ein Implantat 10 in Form eines biventrikulären Dreikammer-Herzschrittmachers und Cardioverters/Defibrillators (ICD). An diesen sind drei Elektrodenleitungen angeschlossen, nämlich eine rechtsatriale Elektrodenleitung 14, eine rechtsventrikuläre Elektrodenleitung 16 und eine linksventrikuläre Elektrodenleitung 30. Im implantierten Zustand endet die rechtsatriale Elektrodenleitung 14 im rechten Atrium 26 eines Herzens 12. Die rechtsventrikuläre Elektrodenleitung 16 endet im rechten Ventrikel 28 des Herzens 12 und die linksventrikuläre Elektrodenleitung 30 reicht über den Coronarsinus des Herzens 12 bis zum linken Ventrikel des Herzens.

Die rechtsatriale Elektrodenleitung 14 trägt an ihrem distalen Ende eine rechtsatriale Spitzenelektrode 22 und in geringem Abstand davon eine rechtsatriale Ringelektrode 24. In ähnlicher Weise trägt die rechtsventrikuläre Elektrodenleitung 16 an ihrem distalen Ende eine rechtsventrikuläre Spitzenelektrode 18 und wenig entfernt davon eine rechtsventrikuläre Ringelektrode 20. Auch am distalen Ende der linksventrikulären Elektrodenleitung 30 ist eine linksventrikuläre Spitzenelektrode 34 und wenig entfernt davon eine linksventrikuläre Ringelektrode 32 angebracht. Diese Elektroden dienen der Aufnahme elektrischer Potenziale in der jeweiligen Herzkammer sowie der Abgabe von Stimulationsimpulsen an die jeweilige Herzkammer im normalen Schrittmacherbetrieb. Diese braucht an dieser Stelle nicht mehr erläutert zu werden.

Die rechtsventrikuläre Elektrodenleitung 16 trägt außerdem noch eine im implantierten Zustand im rechten Ventrikel angeordnete rechtsventrikuläre Schockwendel 38 als Defibrillationselektrode sowie eine im implantierten Zustand in der Vena cava superior befindliche zweite Schockwendel 40. Auch an der linksventrikulären Elektrodenleitung 30 ist eine linksventrikuläre Schockwendel 36 angebracht. Die Schockwendeln dienen im Bedarfsfall als Defibrillations-Elektroden zur Abgabe von Defibrillationsschocks. Auch dies braucht an dieser Stelle nicht näher erläutert zu werden.

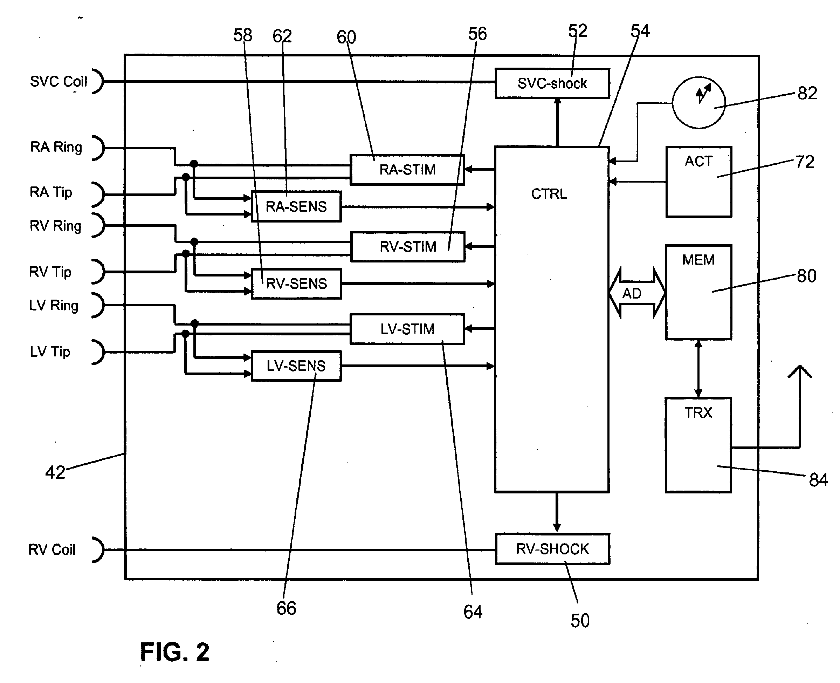

In Figur 2 sind die Hauptbestandteile des Herzstimulators 10 dargestellt. Auf der linken Seite sind die elektrischen Anschlüsse für die verschiedenen Elektroden 18, 20, 24, 22, 32, 34, 36, 38 und 40 dargestellt. Die Schockelektroden 38 und 40 sowie 36 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Spitzenelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode RV Ring die rechtsventrikuläre Spitzenelektrode RV Tip abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten rechten Ventrikel 28 weiter aus und sorgt so für eine stimulierte Kontraktion des rechten Ventrikels 28.

Die rechtsventrikuläre Sensingeinheit 58 ist dazu ausgebildet, an dem Anschluss für die rechtsventrikuläre Ringelektrode RV Ring und die rechtsventrikuläre Tippelektrode RV Tip anliegende elektrische Potentiale zunächst durch einen Eingangsverstärker zu verstärken und zu filtern. Weiterhin ist die rechtsventrikuläre Sensingeinheit 58 ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels 28 detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 28, die durch Schwellwertvergleich detektiert werden kann. Die rechtsventrikuläre Sensingeinheit 58 gibt daraufhin ein entsprechendes, eine natürliche Kontraktion des rechten Ventrikels 28 anzeigendes Ausgangssignal an die Stimulationssteuereinheit 54 aus.

In analoger Weise sind der Anschluss für die rechtsatriale Spitzenelektrode RA Tip und der Anschluss für die rechtsatriale Ringelektrode RA Ring sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 26 kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums 26 kennzeichnet.

In gleicher Weise sind auch der Anschluss für die linksventrikuläre Spitzenelektrode LV Tip und der Anschluss für die linksventrikuläre Ringelektrode LV Ring mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Als weiterer Bestandteil des Herzstimulators 10 ist ein Beschleunigungssensor 72 mit der Stimulationssteuereinheit 54 verbunden und in das Gehäuse 42 des Herzstimulators 10 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an ein externes Gerät zu übertragen oder Programmierbefehle seitens des externen Geräts zu dem Herzstimulator 10 zu übertragen und in der Speichereinheit 80 zu speichern.

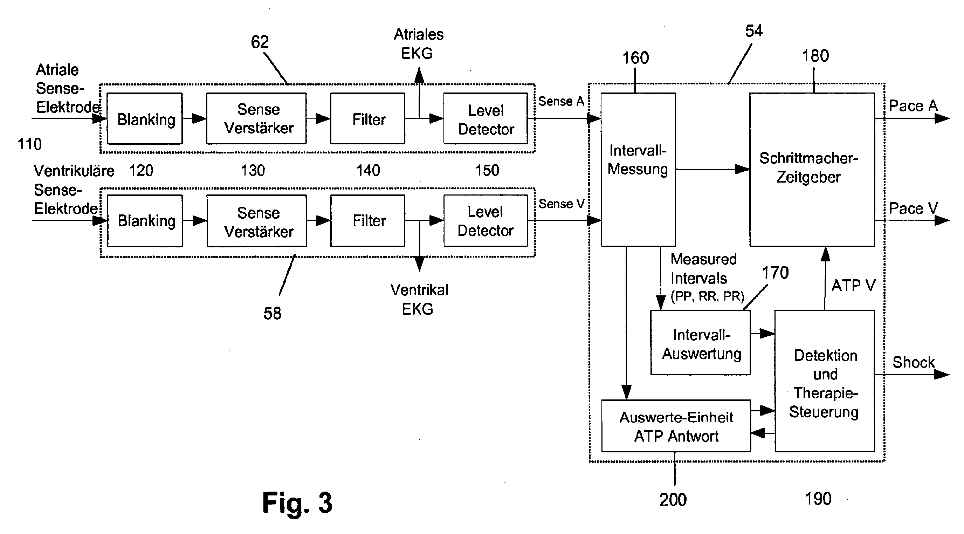

Figur 3 zeigt die für die Erfindung relevanten Komponenten des Herzstimulators 10, nämlich die rechtsatriale Sensingeinheit 62, die rechtsventrikuläre Sensingeinheit 58 und die Stimulationssteuereinheit 54 in detaillierter Darstellung, wobei diese Einheit, insbesondere die Stimulationssteuereinheit 54, noch weitere Bestandteile als die in Figur 3 dargestellten aufweisen oder aufweisen können. Die rechtsatriale Sensingeinheit 62 und die rechtsventrikuläre Sensingeinheit 54 sind jeweils mit einem Anschluss 110 für eine atriale und eine ventrikuläre Elektrode verbunden und weisen jeweils eine Blankingstufe 120 und jeweils einen Verstärker 130 auf, die jeweils in Reihe zueinander dem jeweiligen Elektrodenanschluss 110 nachgeschaltet sind. Die jeweilige Blankingstufe 120 dient in bekannter Manier zur Ausblendung von Stimulationsartefakten nach Abgabe eines Stimulationsimpulses. Durch den jeweiligen Verstärker 130 werden die mittels atrialer bzw. ventrikulärer Elektrode abgeleiteten intrakardialen Signale zunächst verstärkt. Auf den jeweiligen Verstärker 130 folgt jeweils eine A/D-Wandler- und Filterstufe 140, die das Signal digitalisiert und filtert. Auf die A/D-Wandler- und Filterstufe 140 folgt jeweils ein Komparator 150, der das digitalisierte und gefilterte Signal jeweils mit einem Schwellwert vergleicht, um im Falle des intraatrialen Signals durch Schwellwertvergleich P-Wellen zu detektieren und im Falle des intraventrikulären Signals durch Schwellwertvergleich R-Wellen zu detektieren. Auf diese Weise sind die rechtsatriale Sensingeinheit 62 und die rechtsventrikuläre Sensingeinheit 58 jeweils in der Lage, ein rechtsatriales bzw. ein rechtsventrikuläres Sense-Signal zu erzeugen, falls eine Kontraktion der jeweiligen Herzkammer vorliegt.

Die so gewonnen P- und R-Wellen synchronen Ereignisse (Sense-Signale) werden der Stimulationssteuereinheit 54 zugeführt und dort von einer Intervallmesseinheit 160 hinsichtlich der zwischen ihnen auftretenden Intervalle ausgewertet. Diese so gewonnenen PP-, PR-, RP- und RR-Intervalle werden einem Schrittmacherzeitgeber 180 zur Steuerung der antibradykarden Stimulation zugeführt. Ferner werden diese Intervallinformationen in einer Intervallauswerteeinheit 170 zur Diskriminierung zwischen VT und SVT hinsichtlich ihrer Verhältnisse bezüglich atrialem und ventrikulärem Rhythmus verglichen und in bewerteter Form einer Detektions- und Therapiesteuereinheit 190 für die antitachykarde Therapie (ATP, Schock) verfügbar gemacht.

Erfindungsgemäß weist der Herzstimulator z.B. als Bestandteil der Stimulationssteuereinheit 54 zusätzlich eine Auswerteinheit 200 zur Tachykardiediskriminierung auf, die mit der Intervallmesseinheit 160 und der Detektions- und Therapiesteuereinheit 190 verbunden ist und die ausgebildet ist, ein ATP-Antwortsignal zu erzeugen.

Dazu wertet die Auswerteeinheit 200 die in der Intervallmesseinheit 160 bestimmten Intervalle unmittelbar vor- und während einer ATP aus. Zu diesem Zweck besteht eine bidirektionale Verbindung zwischen der Auswerteeinheit 200 und der Therapiesteuereinheit 190. Die Auswerteeinheit 200 wird von der Therapiesteuereinheit 190 über die bevorstehende ATP-Abgabe informiert und beeinflusst mit dem jeweils gewonnen ATP-Antwortsignal wiederum die Therapiesteuereinheit 190. Die Bestimmung des ATP-Antwortsignals erfolgt dabei durch einen Intervall- oder Mustervergleich der atrialen Intervalle vor und während einer ventrikulären ATP. Bleibt dabei der atriale Rhythmus von der ventrikulären ATP unbeeinflusst (=), so wird eine SVT angenommen. Wird der atriale Rhythmus von der ventrikulären ATP moduliert (≠), so wird eine VT bestätigt. Die Bewertung ist dabei derart gestaltet, dass durch Interpolation diejenigen atrialen Intervalle ersetzt werden, die ggf. durch ein Cross-Blank während ventrikulärer ATP ausgeblendet wurden.

In den nachfolgenden Abbildungen sind mögliche Abläufe für die Therapieablaufsteuerung durch die Therapiesteuereinheit 190 in Abhängigkeit des ATP-Antwortsignals dargestellt.

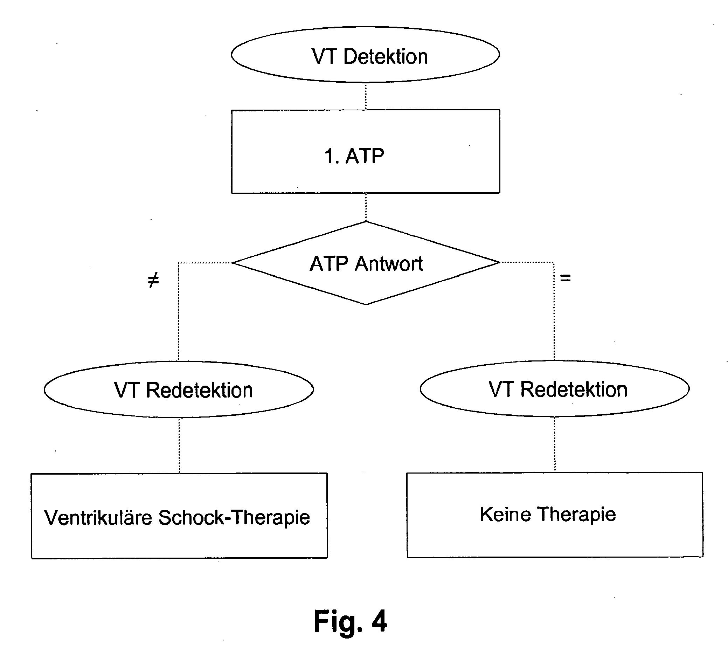

Die Abbildung 4 zeigt dabei eine einfache Erweiterung der Therapieablaufsteuerung mittels des ATP-Antwortsignals: Nachdem eine VT-Detektion (bei einem Rhythmus mit gleichzeitig schnellem Atrium) erfüllt wurde, wird zunächst die programmierte antitachykarde Stimulation abgegeben und zugleich das ATP-Antwortsignal bestimmt. Wird der atriale Rhythmus von der ventrikulären ATP moduliert, dann folgt der ATP nach erfüllter VT-Redetektion eine programmierte ventrikuläre Schocktherapie, andernfalls wird trotz erfüllter Redetektion keine Schocktherapie freigegeben, da in diesem Falle die initiale VT-Klassifikation aufgrund des ATP-Antwortsignals zugunsten einer SVT korrigiert wurde.

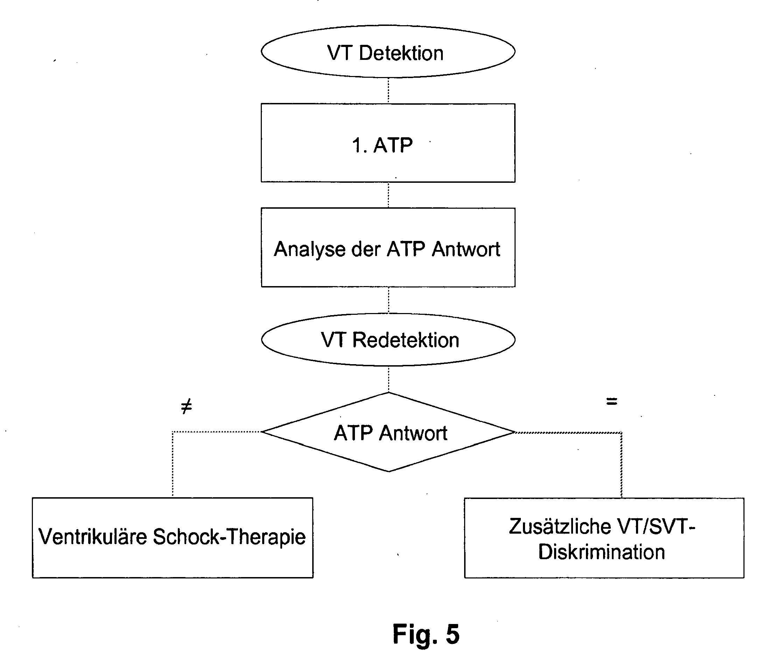

Eine alternative Implementierung ist in Abbildung 5 dargestellt: Hier erfolgt nach initialer VT-Detektion eine ATP und gleichzeitige Analyse des ATP-Antwortsignals. Wird nach der ATP eine erneute VT-Redetektion erfüllt, dann wird in Abhängigkeit des ATP-Antwortsignals entweder eine ventrikuläre Schocktherapie abgegeben oder aber die VT/SVT-Diskriminierung erneut gestartet. So ist es möglich, eine kurze Redetektionszeit zwischen ATP und Schocktherapie zu gewährleisten und nur bei "zweifelhafter" VT-Klassifikation eine verlängerte Redetektion mit erweiterten Diskriminierungskriterien (z. B. verlängerte Detektionszähler) zu starten.

Figur 6:

Anders als in Figur 4 wird hier erfindungsgemäß im Falle eines ATP-Antwortsignals, das eine SVT repräsentiert, nach Redetektion entschieden, ob die atriale Arrhythmie stabil oder instabil ist und im Falle eines Vorhofflimmerns (instabil) eine atriale Therapie (HF-Burst oder Kardioversionsschock) eingeleitet. Ist der atriale Rhythmus hingegen stabil, erfolgt keine Therapieabgabe (belastungsinduzierter Frequenzanstieg).

## Patentansprüche

1. Implantierbarer Herzstimulator, mit
- einer ersten Kammerstimulationseinheit (56; 64), die mit einer linksventrikulären oder einer rechtsventrikulären Stimulationselektrode verbunden oder zu verbinden und ausgebildet ist, Kammerstimulationsimpulse zur Stimulation eines Ventrikels eines Herzens zu generieren und abzugeben,
- einer zweiten Kammerstimulationseinheit (60), die mit einer atrialen Stimulationselektrode verbunden oder zu verbinden und ausgebildet ist, Kammerstimulationsimpulse zur Stimulation eines Atriums eines Herzens zu generieren und abzugeben,
- einer ventrikulären Sensingeinheit (58; 66), die ausgebildet ist, eine jeweilige Kammerkontraktion zu erfassen und im Falle einer detektierten Kammerkontraktion ein ventrikuläres Sensingsignal auszugeben,
- einer atrialen Sensingeinheit (62), die ausgebildet ist, eine Vorhofkontraktion zu erfassen und im Falle einer detektierten Vorhofkontraktion ein ein jeweiliges atriales Ereignis anzeigendes atriales Sensingsignal auszugeben,
- einer Tachykardieerfassungseinheit (170), die wenigstens mit einer ventrikulären Sensingeinheit verbunden und ausgebildet ist, eine Tachykardie zu erfassen und als ventrikuläre Tachykardie, VT, oder als superventrikuläre Tachykardie, SVT, zu kategorisieren, und
- einer Therapiesteuereinheit (190), die ausgebildet ist, die erste Kammerstimulationseinheit (56; 64) zur Abgabe einer ventrikulären antitachykarden Stimulation, ATP, und die zweite Kammerstimulationseinheit (60) zur Abgabe einer atrialen antitachykarden Stimulation anzusteuern,
- einer Auswerteeinheit (200), die mit der atrialen Sensingeinheit (62) und der Therapiesteuereinheit verbunden und ausgebildet ist, von der atrialen Sensingeinheit (62) vor, während und nach einer Abgabe einer antitachykarden Stimulation erfasste atriale Ereignisse hinsichtlich des atrialen Rhythmusmusters während ventrikulärer ATP mit dem atrialen Rhythmusmuster unmittelbar vor der ATP zu vergleichen und die Therapiesteuereinheit (190) in Abhängigkeit eines das Vergleichsergebnis repräsentierenden ATP-Antwortsignals zur Auswahl der nachfolgenden antitachykarden Therapie anzusteuern,
wobei die Auswerteeinheit (200) ausgebildet ist, ein eine superventrikuläre Tachykardie anzeigendes ATP-Antwortsignal zu generieren, wenn der Vergleich der atrialen Rhythmusmuster ergibt, dass der atriale Rhythmus von der ventrikulären ATP unbeeinflusst bleibt und eine ventrikuläre Tachykardie zu bestätigen und ein eine ventrikuläre Tachykardie anzeigendes ATP-Antwortsignal zu generieren, wenn der Vergleich der atrialen Rhythmusmuster ergibt, dass der atriale Rhythmus von der ventrikulären ATP moduliert wird
**dadurch gekennzeichnet dass**
die Therapiesteuereinheit (190) ausgebildet ist, im Falle eines ATP-Antwortsignals, das eine SVT repräsentiert, zu entscheiden, ob der atriale Rhythmus stabil oder instabil ist und im Falle eines instabilen atrialen Rhythmus eine atriale antitachykarde Stimulationstherapie einzuleiten, und im Falle eines stabilen atrialen Rhythmus keine Therapieabgabe zu veranlassen.

2. Implantierbarer Herzstimulator nach Anspruch 1, **dadurch gekennzeichnet, dass** die atriale antitachykarde Stimulationstherapie eine atriale Kardioversionsschocktherapie ist.

3. Implantierbarer Herzstimulator nach Anspruch 1, **dadurch gekennzeichnet, dass** die Kammersensingeinheit (58; 66) mit einer linksventrikulären oder einer rechtsventrikulären Sensingelektrode verbunden oder zu verbinden und ausgebildet ist, elektrische Potentiale einer jeweiligen Herzkammer zu erfassen und hinsichtlich einer Kammerkontraktion auszuwerten.

4. Implantierbarer Herzstimulator nach Anspruch 1, **dadurch gekennzeichnet, dass** die Vorhofsensingeinheit (62) mit einer atrialen Sensingelektrode verbunden oder zu verbinden und ausgebildet ist, elektrische Potentiale des vorzugsweise rechten Vorhofs eines Herzens zu erfassen.

5. Implantierbarer Herzstimulator nach einem der Ansprüche 1 bis 4, **dadurch gekennzeichnet, dass** die Tachykardieerfassungseinheit (170) ausgebildet ist, einen jeweils aktuellen ventrikulären Rhythmus mindestens einer VT- und einer VF-Zone zuzuordnen.

6. Implantierbarer Herzstimulator nach einem der Ansprüche 1 bis 5, **gekennzeichnet durch** einen Defibrillationsschockgenerator (50), der mit einer Defibrillationsschockelektrode (38) verbunden oder zu verbinden und ausgebildet ist, einen ventrikulären Defibrillationschock zur Defibrillation wenigstens einer Herzkammer zu generieren und auszugeben.

7. Implantierbarer Herzstimulator nach Anspruch 6, **dadurch gekennzeichnet, dass** die Therapiesteuereinheit (190) ausgebildet ist, die Abgabe eines ventrikulären Defibrillationsschocks zu inhibieren, wenn das ATP-Anwortsignal ein Vorliegen einer superventrikulären Tachykardie anzeigt.

8. Implantierbarer Herzstimulator nach Anspruch 5 und 7, **dadurch gekennzeichnet, dass** die Therapiesteuereinheit (190) ausgebildet ist, die Abgabe eines ventrikulären Defibrillationsschocks zu inhibieren, wenn das ATP-Anwortsignal ein Vorliegen einer superventrikulären Tachykardie anzeigt und die jeweils zugehörige ventrikuläre Herzrate in einer VT-Zone liegt.

9. Implantierbarer Herzstimulator nach einem der Ansprüche 1 bis 8, **dadurch gekennzeichnet, dass** die Therapiesteuereinheit (190) ausgebildet ist, eine nachfolgende antitachykarde Therapie in Abhängigkeit des ATP-Antwortsignals zu inhibieren.

10. Implantierbarer Herzstimulator nach einem der Ansprüche 1 bis 8, **dadurch gekennzeichnet, dass** die Therapiesteuereinheit (190) ausgebildet ist, eine nachfolgende antitachykarde Therapie in Abhängigkeit des ATP-Antwortsignals fortzusetzen.

11. Implantierbarer Herzstimulator nach einem der Ansprüche 1 bis 8, **dadurch gekennzeichnet, dass** die Therapiesteuereinheit (190) ausgebildet ist, in Abhängigkeit des ATP-Antwortsignals zunächst eine neue VT/SVT Klassifikation zu starten.

12. Implantierbarer Herzstimulator nach einem der Ansprüche 1 bis 8, **dadurch gekennzeichnet, dass** die Therapiesteuereinheit (190) ausgebildet ist, für eine nachfolgende antitachykarde Therapie in Abhängigkeit des ATP-Antwortsignals die Parameter der ventrikulären antitachykarden Therapie anzupassen.

## Claims

1. An implantable cardiac stimulator, comprising
- a first chamber stimulation unit (56; 64), which is connected or is to be connected to a left-ventricular or a right-ventricular stimulation electrode and is designed to generate and to deliver chamber stimulation pulses for stimulation of a ventricle of a heart,
- a second chamber stimulation unit (60), which is connected or is to be connected to an atrial stimulation electrode and is designed to generate and to deliver chamber stimulation pulses for stimulation of an atrium of a heart,
- ventricular sensing unit (58; 66), which is designed to detect a respective chamber contraction and to deliver a ventricular sensing signal when a chamber contraction is detected,
- an atrial sensing unit (62), which is designed to detect an atrial contraction and to deliver an atrial sensing signal that indicates a respective atrial event when an atrial contraction is detected,
- a tachycardia detection unit (170), which is connected at least to one ventricular sensing unit and is designed to detect a tachycardia and to categorise the tachycardia as a ventricular tachycardia VT or as a supraventricular tachycardia SVT,
- a treatment control unit (190), which is designed to actuate the first chamber stimulation unit (56; 64) to deliver a ventricular antitachycardiac stimulation ATP and to actuate the second chamber stimulation unit (60) to deliver an atrial antitachycardiac stimulation,
- an evaluation unit (200), which is connected to the atrial sensing unit (62) and to the treatment control unit and is designed to compare atrial events detected by the atrial sensing unit (62) before, during and after delivery of an antitachycardiac stimulation with regard to the atrial rhythm pattern during ventricular ATP with the atrial rhythm pattern immediately before ATP and to actuate the treatment control unit (190) depending on an ATP response signal that represents the result of the comparison for selection of the subsequent antitachycardiac treatment,
wherein the evaluation unit (200) is designed to generate an ATP response signal that indicates a supraventricular tachycardia when the comparison of the atrial rhythm pattern reveals that the atrial rhythm remains uninfluenced by the ventricular ATP, and to confirm a ventricular tachycardia and to generate an ATP response signal that indicates a ventricular tachycardia when the comparison of the atrial rhythm pattern reveals that the atrial rhythm is modulated by the ventricular ATP,
**characterised in that**
the treatment control unit (190) is designed, in the case of an ATP response signal that represents an SVT, to decide whether the atrial rhythm is stable or unstable, and, in the case of an unstable atrial rhythm, to commence atrial antitachycardiac stimulation treatment, and, in the case of a stable atrial rhythm, to initiate no delivery of treatment.

2. The implantable cardiac stimulator according to Claim 1, **characterised in that** the atrial antitachycardiac stimulation treatment is atrial cardioversion shock treatment.

3. The implantable cardiac stimulator according to Claim 1, **characterised in that** the chamber sensing unit (58; 66) is connected or is to be connected to a left-ventricular or a right-ventricular sensing electrode and is designed to detect electric potentials of a respective heart chamber and to evaluate the electric potentials with respect to a chamber contraction.

4. The implantable cardiac stimulator according to Claim 1, **characterised in that** the atrial sensing unit (62) is connected or is to be connected to an atrial sensing electrode and is designed to detect electric potentials preferably of the right atrium of the heart.

5. The implantable cardiac stimulator according to one of Claims 1 to 4, **characterised in that** the tachycardia detection unit (170) is designed to assign a current ventricular rhythm to at least one VT zone and one VF zone.

6. The implantable cardiac stimulator according to one of Claims 1 to 5, **characterised by** a defibrillation shock generator (50), which is connected or is to be connected to a defibrillation shock electrode (38) and is designed to generate and to deliver a ventricular defibrillation shock for defibrillation of at least one heart chamber.

7. The implantable cardiac stimulator according to Claim 6, **characterised in that** the treatment control unit (190) is designed to inhibit the delivery of a ventricular defibrillation shock when the ATP response signal indicates occurrence of a supraventricular tachycardia.

8. The implantable cardiac stimulator according to Claims 5 and 7, **characterised in that** the treatment control unit (190) is designed to inhibit the delivery of a ventricular defibrillation shock when the ATP response signal indicates occurrence of a supraventricular tachycardia and the respective ventricular heart rate is in a VT zone.

9. The implantable cardiac stimulator according to one of Claims 1 to 8, **characterised in that** the treatment control unit (190) is designed to inhibit a subsequent antitachycardiac treatment depending on the ATP response signal.

10. The implantable cardiac stimulator according to one of Claims 1 to 8, **characterised in that** the treatment control unit (190) is designed to continue a subsequent antitachycardiac treatment depending on the ATP response signal.

11. The implantable cardiac stimulator according to one of Claims 1 to 8, **characterised in that** the treatment control unit (190) is designed to first start a new VT/SVT classification depending on the ATP response signal.

12. The implantable cardiac stimulator according to one of Claims 1 to 8, **characterised in that** the treatment control unit (190) is designed to adapt for a subsequent antitachycardiac treatment depending on the ATP response signal the parameters of the ventricular antitachycardiac treatment.

## Revendications

1. Stimulateur cardiaque implantable, avec
- une première unité de stimulation ventriculaire (56 ; 64), reliée ou destinée à être reliée à une électrode de stimulation du ventricule gauche ou du ventricule droit, et conçue pour générer et délivrer des impulsions de stimulation ventriculaire pour la stimulation d'un ventricule d'un coeur,
- une deuxième unité de stimulation ventriculaire (60) reliée ou destinée à être reliée à une électrode de stimulation atriale et conçue pour générer et délivrer des impulsions de stimulation ventriculaire,
- une unité de détection ventriculaire (58 ; 66) conçue pour détecter une contraction ventriculaire respective et pour délivrer un signal de détection ventriculaire en cas de détection d'une contraction ventriculaire,
- une unité de détection atriale (62) conçue pour détecter une contraction ventriculaire et détecter une contraction atriale et pour délivrer un signal de détection atriale indiquant un évènement atrial respectif en cas de détection d'une contraction atriale,
- une unité de détection de tachycardie (170) reliée au moins à une unité de détection ventriculaire et conçue pour détecter une tachycardie et pour classer celle-ci comme une tachycardie ventriculaire VT ou comme une tachycardie superventriculaire SVT, et
- une unité de commande thérapeutique (190) conçue pour commander la première unité de stimulation ventriculaire (56 ; 64) en vue de la délivrance d'une stimulation ventriculaire antitachycardiaque ATP et pour commander la deuxième unité de stimulation ventriculaire (60) en vue de la délivrance d'une stimulation atriale antitachycardiaque,
- une unité d'évaluation (200) reliée à l'unité de détection atriale (62) et à l'unité de commande thérapeutique, et conçue pour comparer les évènements atriaux détectés par l'unité de détection atriale (62) avant, pendant et après une délivrance d'une stimulation antitachycardiaque, concernant le modèle de rythme atrial pendant l'ATP ventriculaire, avec le modèle de rythme atrial, directement avant l'ATP, et pour actionner l'unité de commande thérapeutique (190) en fonction d'un signal de réponse ATP représentant le résultat de la comparaison, en vue de la sélection de la thérapie antitachycardiaque consécutive,
dans lequel l'unité d'évaluation (200) est conçue pour générer un signal de réponse ATP indiquant une tachycardie superventriculaire lorsque la comparaison des modèles de rythme atrial montrent que le rythme atrial de l'ATP ventriculaire n'est pas influencé, et pour confirmer une tachycardie ventriculaire et générer un signal de réponse ATP indiquant une tachycardie ventriculaire lorsque la comparaison des modèles de rythme atrial montrent que le rythme atrial est modulé par l'ATP ventriculaire,
**caractérisé en ce que**
l'unité de commande thérapeutique (190) est conçue pour déterminer, en cas de signal de réponse ATP représentant une SVT, si le rythme atrial est stable ou instable, et pour initier une thérapie de stimulation atriale antitachycardiaque en cas de rythme atrial instable, et pour ne pas engager de délivrance thérapeutique en cas de rythme atrial stable.

2. Stimulateur cardiaque implantable selon la revendication 1, **caractérisé en ce que** la thérapie de stimulation atriale antitachycardiaque est une thérapie de choc de cardioversion atriale.

3. Stimulateur cardiaque implantable selon la revendication 1, **caractérisé en ce que** l'unité de détection ventriculaire (58 ; 66) est reliée ou destinée à être reliée à une électrode de détection du ventricule gauche ou du ventricule droit, et conçue pour détecter des potentiels électriques d'un ventricule respectif et évaluer ceux-ci en vue d'une contraction ventriculaire.

4. Stimulateur cardiaque implantable selon la revendication 1, **caractérisé en ce que** l'unité de détection atriale (62) est reliée ou destinée à être reliée à une électrode de détection atriale, et conçue pour détecter des potentiels électriques de l'oreillette d'un coeur, de préférence celle de droite.

5. Stimulateur cardiaque implantable selon l'une des revendications 1 à 4, **caractérisé en ce que** l'unité de détection de tachycardie (170) est conçue pour attribuer un rythme ventriculaire respectivement actuel à au moins une zone VT et une zone VF.

6. Stimulateur cardiaque implantable selon l'une des revendications 1 à 5, **caractérisé par** un générateur de chocs de défibrillation (50) relié ou destiné à être relié à une électrode de choc de défibrillation (38), et conçu pour générer et délivrer un choc de défibrillation ventriculaire, en vue de la défibrillation d'au moins un ventricule.

7. Stimulateur cardiaque implantable selon la revendication 6, **caractérisé en ce que** l'unité de commande thérapeutique (190) est conçue pour empêcher la délivrance d'un choc de défibrillation ventriculaire lorsque le signal de réponse ATP indique l'existence d'une tachycardie superventriculaire.

8. Stimulateur cardiaque implantable selon les revendications 5 et 7, **caractérisé en ce que** l'unité de commande thérapeutique (190) est conçue pour empêcher la délivrance d'un choc de défibrillation ventriculaire lorsque le signal de réponse ATP indique l'existence d'une tachycardie superventriculaire et que le rythme cardiaque ventriculaire correspondant se trouve dans une zone VT.

9. Stimulateur cardiaque implantable selon l'une des revendications 1 à 8, **caractérisé en ce que** l'unité de commande thérapeutique (190) est conçue pour empêcher une thérapie antitachycardiaque consécutive en fonction du signal de réponse ATP.

10. Stimulateur cardiaque implantable selon l'une des revendications 1 à 8, **caractérisé en ce que** l'unité de commande thérapeutique (190) est conçue pour poursuivre une thérapie antitachycardiaque consécutive en fonction du signal de réponse ATP.

11. Stimulateur cardiaque implantable selon l'une des revendications 1 à 8, **caractérisé en ce que** l'unité de commande thérapeutique (190) est conçue pour démarrer tout d'abord une nouvelle classification VT/SVT en fonction du signal de réponse ATP.

12. Stimulateur cardiaque implantable selon l'une des revendications 1 à 8, **caractérisé en ce que** l'unité de commande thérapeutique (190) est conçue pour adapter les paramètres de la thérapie ventriculaire antitachycardiaque d'une thérapie antitachycardiaque consécutive en fonction du signal de réponse ATP.
